(19) Europäisches Patentamt
European Patent Office
Office européen des brevets

(11) Veröffentlichungsnummer: **0 021 310**
**B1**

(12) # EUROPÄISCHE PATENTSCHRIFT

(45) Veröffentlichungstag der Patentschrift: **20.07.83**

(51) Int. Cl.³: **C 12 Q 1/54,** C 12 Q 1/48, G 01 N 33/02

(21) Anmeldenummer: **80103353.1**

(22) Anmeldetag: **16.06.80**

(54) **Verfahren und Reagens zur Bestimmung von Fructose.**

(30) Priorität: **25.06.79 DE 2925534**

(43) Veröffentlichungstag der Anmeldung: **07.01.81 Patentblatt 81/1**

(45) Bekanntmachung des Hinweises auf die Patenterteilung: **20.07.83 Patentblatt 83/29**

(84) Benannte Vertragsstaaten: **AT BE CH DE FR GB IT LI LU NL SE**

(56) Entgegenhaltungen:
**Chemical Abstracts, Band 49, Nr. 14, 25. Juni 1955, Spalte 9756h Columbus, Ohio, USA C.E. Cardini et al.: «The biosynthesis of sucrose»**
**Chemical abstracts, Band 74, Nr. 17, 16. April 1971, Seite 41, Nr. 83366a Columbus, Ohio, USA R.N. Shukla et al.: «UDP-glucose: D-fructose 2-glucoxyltransferase from tapioca tuber»**
**H. U. Bergmeyer: «Methoden der Enzymatischen Analyse», 3. Auflage 1974, Band 2, Verlag Chemie Seiten 1194, 1195, 1221, 1349–1352; 2199–2202 Weinheim, DE**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Beutler, Hans-Otto, Dr., Zugspitzstrasse 28, D-8132 Tutzing (DE)**
Erfinder: **Michal, Gerhard, Dr., Kreuzeckstrasse 19, D-8132 Tutzing (DE)**
Erfinder: **Stähler, Fritz, Dr., Heimgartenstrasse 4, D-8132 Tutzing (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

**EP 0 021 310 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren und Reagens zur Bestimmung von Fructose

Die Erfindung betrifft ein Verfahren und ein Reagens zur Bestimmung von Fructose und Fructose enthaltenden Glykosiden, insbesondere in Gegenwart von im Überschuss vorliegenden anderen Kohlehydraten.

Die Bestimmung von Kohlehydraten gehört in der Lebensmittelchemie zu den elementaren analytischen Aufgaben. Es gibt eine grosse Zahl von analytischen Methoden, welche zur Ermittlung der Kohlehydratzusammensetzung angewendet werden. Eine Zusammenfassung findet sich im «Handbuch der Lebensmittelchemie» II/2 (1967), Springer-Verlag. Die bekannten Analysenverfahren weisen jedoch den Nachteil einer sehr aufwendigen und umständlichen Durchführung auf, wenn es gilt, nicht nur die Summe aller Kohlehydrate – meist über physikalische oder physikochemische Verfahren – zu ermitteln (z.B. Extraktgehalt über Refraktometrie, Dichtebestimmung oder Polarimetrie), sondern auch eine Selektivierung der verschiedenen Kohlehydratbestandteile anzustreben.

Die wichtigsten Zucker in der Lebensmittelchemie sind Glucose, Fructose und Saccharose. Wegen des Mangels an chemischen Methoden zur Bestimmung von Glucose und Fructose nebeneinander wird im Bereich der Lebensmittelchemie zumeist die Summe beider Zucker bestimmt und dieser Messwert als «Reduktionszucker» oder «reduzierende Zucker» zur Beurteilung von Lebensmitteln herangezogen. Ausdruck dieser analytischen Schwäche sind sehr viele Lebensmittelgesetze und Verordnungen, in denen gerade diese Zuckerformen verankert sind (Weingesetz, Fruchtsaft-Verordnung, Verordnung über Obsterzeugnisse).

In vielen Fällen ist die Bestimmung des Extraktgehalts (über Trockenverlust bestimmt) durchaus repräsentativ für den Gehalt an reduzierenden Zuckern oder man bedient sich der Luff/Schoorl-Methode [Chem. Weekbl. 9, 678, 706 (1912); ZUL 57, 566 (1929)], mit der man die reduzierenden Eigenschaften der Monosaccharide zur Gehaltsbestimmung auswerten kann.

Sehr störanfällig wird dieses Verfahren jedoch dann, wenn auch andere reduzierende Komponenten im Lebensmittel vorliegen [Vitamin C in Fruchtsäften, Galacturonsäure, Reduktone in Wein; siehe G. Baumann und K. Gierschner, Ind. Obst- und Gemüseverwertung, 56, 165 (1971)]. In solchen Fällen wird eine spezifische Bestimmung der Glucose und der Fructose unerlässlich. Zwar sind auch Methoden bekannt, um eine solche Differenzierung vornehmen zu können (z.B. alkalische Jod-Methode), doch sind diese störanfällig.

Eine wesentliche Verbesserung bei der Fructosebestimmung brachte die Anwendung einer enzymatischen Methode, welche in den letzten Jahren immer stärkere Verbreitung fand. Sie beruht auf der Phosphorylierung der Fructose mit ATP in Gegenwart von Hexokinase und Phosphoglucoseisomerase und Bildung von Glucose-6-phosphat und dessen Bestimmung mit seiner Dehydrogenase und NADP. Aber auch diese Methode ist in der einfachen Form nur dann anwendbar, wenn die Konzentrationen von Glucose und Fructose in Verhältnissen von 1:0,03 bis 1:30 vorliegen. Ausserhalb dieses Bereichs liegende Konzentrationsverhältnisse zwingen auch bei Anwendung der enzymatischen Methodik dazu, ergänzende Techniken anzuwenden, z.B. die Beseitigung einer Zuckerkomponente. Überschüssige Glucose kann neben geringen Mengen Fructose mit Hilfe von Gluoseoxidase (GOD) völlig entfernt werden, so dass die anschliessende Messung von Fructose möglich wird (H.U. Bergmeyer, «Methoden der enzymatischen Analyse» 1974, Verlag-Chemie, Weinheit, S. 1349). Bei extremen Überschüssen von Glucose neben geringen Fructosekonzentrationen dagegen ist eine völlige Entfernung der Glucose nicht möglich (H.O. Beutler, G. Michal, G. Beinstingl: Deutsche Lebensm. Rundschau 1978). In diesen Fällen kann die Glucosekonzentration bei Anwendung des GOD-Verfahrens lediglich auf eine Restkonzentration gesenkt werden, wonach dann eine messfähige Konzentration beider Zuckerkomponenten resultiert.

Auch die Ermittlung des Gehalts an anderen Fructoseeinheiten enthaltenden Kohlehydraten, wie z.B. Saccharose (Glucosylfructose) bereitet Schwierigkeiten, wenn ungünstige Zuckerverhältnisse vorliegen. Im allgemeinen kann Saccharose durch ein polarimetrisches Verfahren hinreichend spezifisch bestimmt werden (optischer Drehwert einer Lösung vor und nach Inversion). Dieses Verfahren versagt aber in Gegenwart hoher Konzentrationen anderer Zucker oder von Polysacchariden. So ist es nicht möglich, mit jener Methode kleine Saccharosekonzentrationen neben hohen Anteilen von Stärke in Kindernahrungsmitteln oder diätetischen Lebensmitteln zu bestimmen. Hier würde die enzymatische Methode unter Verwendung von β-Fructosidase zur Saccharosemessung geeignet sein. Dieses Verfahren wiederum ist wenig anwendbar, wenn im Lebensmittel ausserdem hohe Konzentrationen von Malzextrakt und Glucose vorliegen. Bei ungünstigen Verhältnissen von Saccharose und anderen Kohlehydraten muss daher – auch bei Anwendung enzymatischer Messtechniken – eine aufwendige Probenvorbereitung vorangehen, bevor die Saccharose bestimmt werden kann.

In der klinischen Chemie wird zu bestimmten diagnostischen Zwecken die Ermittlung der Fructosekonzentration nützlich sein. Da diese Konzentration im Blut sehr niedrig gegenüber der gleichzeitig hohen Konzentration von Glucose vorliegt, kann eine Messung jener Konzentration nur mit hohem Aufwand erfolgen.

Bei der Inulin-Clearance ist die Bestimmung von Inulin im Blut wünschenswert. Nach Hydrolyse des Inulins (Polyfructose, Fructosan) durch Säurebehandlung oder durch Inulinase erhält man das Hydrolyseprodukt Fructose, aus dessen

Konzentration auf den Inulingehalt geschlossen werden kann.

Der Erfindung liegt daher die Aufgabe zugrunde, ein Verfahren zur Bestimmung von Fructose zu schaffen, welches die obigen Nachteile nicht aufweist und sich in Gegenwart von im Überschuss vorliegenden anderen Kohlehydraten, wie Zuckern oder Polysacchariden, durchführen lässt. Dieses Verfahren soll auch zur Bestimmung von aus anderen Kohlehydraten freigesetzter Fructose geeignet sein und damit die Bestimmung derartiger fructosehaltiger Kohlehydrate ermöglichen.

Gelöst wird diese Aufgabe erfindungsgemäss durch ein Verfahren zur Bestimmung von Fructose in Gegenwart von im Überschuss vorliegenden anderen Zuckern oder Polysacchariden und zur Bestimmung von Fructose enthaltenden Glykosiden, insbesondere in Gegenwart von im Überschuss vorliegenden anderen Zuckern oder Polysacchariden, welches dadurch gekennzeichnet ist, dass die gegebenenfalls zuerst aus dem Glykosid in Freiheit gesetzte Fructose mit UDPG und Saccharosesynthetase umgesetzt wird unter Bildung von UDP, welches in an sich bekannter Weise bestimmt wird.

Überraschenderweise gelingt es erfindungsgemäss, Fructose und fructosehaltige Glykoside unabhängig von der Konzentration anderer Zucker oder Kohlehydrate zu bestimmen. Beispielsweise wurde gefunden, dass auch in grossem Überschuss vorliegende Cellulose, Pentosane, Hemicellulosen, Mannane, Calactane, Inulin, Laevan, Raffinose, Saccharose, Lactose, Maltose, Glucosephosphat, Mannose, Mannosephosphat, Mannit, Mannitphosphat, Galactose, Galactosephosphat, Fructosephosphat, Sorbit, Sorbitphosphat, Inosith, Pentosen, wie Ribose, Arabinose und Xylose, Pentosephosphat, Erythrit und Erythritphosphat nicht stören. Dies ist um so überraschender, als nach Angaben der Literatur auch andere Kohlehydrate, wie z.B. Fructose-6-phosphat, von der Saccharosesynthetase umgesetzt werden. Ebenso erstaunlich ist, dass die Fructosebestimmung auch durch einen hohen Überschuss an Saccharose, welche ja Reaktionsprodukt ist, nicht gestört wird.

Im übrigen war durchaus nicht vorhersehbar, dass die Saccharosesynthease sich überhaupt zur Fructosebestimmung eignen würde, zumal dieses Enzym schon seit mehr als 20 Jahren bekannt ist, ohne dass man eine solche Verwendung bisher in Betracht gezogen hat.

Für die Messung des bei der Reaktion neben Saccharose gebildeten UDP kommen verschiedene Methoden in Betracht. Bevorzugt wird die an sich bekannte Umsetzung von UDP mit Phosphoenolpyruvat (PEP) in Gegenwart von Pyruvatkinase (PK) und Lactatdehydrogenase (LDH) sowie NADH unter Bildung von NAD. Dieses Verfahren lässt sich durch folgende Reaktonsgleichungen veranschaulichen:

$$(1)\ UDP + PEP \xrightarrow{PK} UTP + Pyruvat$$

$$(2)\ Pyruvat + NADH + H^{+} \xrightarrow{LDH} Lactat + NAD^{+}$$

Die photometrisch messbare Umwandlung von NADH zu NAD ist proportional der Fructosekonzentration. Andere Möglichkeiten der UDP-Bestimmung beruhen auf der Phosphorylierung mit einem Phosphatdonator, wie ATP, in Gegenwart der entsprechenden Kinase und Messung des entphosphorylierten Phosphatdonators, wie ADP, nach bekannten Methoden. Auch gebildetes UTP kann gemessen werden.

Eine weitere, auf diesem Prinzip beruhende Ausführungsform besteht in der Phosphorylierung mit Formylphosphat in Gegenwart von Formiatkinase (E.C. 2.7.2.6) unter Bildung von Ameisensäure, welche beispielsweise wiederum mit Formiatdehydrogenase (E.C. 1.2.1.2) in Gegenwart von NAD umgesetzt werden kann unter Bildung von NADH, welches in üblicher Weise gemessen wird.

Ein anderes, auf diesem Prinzip beruhendes Beispiel besteht in der Phosphorylierung mit Arginylphosphat in Gegenwart von Argininkinase (E.C. 2.7.3.3), Spaltung des gebildeten freien Arginins mit Arginase (E.C. 3.5.3.1) unter Bildung von Harnstoff, der in üblicher Weise bestimmt wird, beispielsweise durch Spaltung mit Urease (E.C. 3.5.1.5) unter Bildung von Ammoniak, welcher gemessen wird.

Das erfindungsgemässe Verfahren wird, wie bei enzymatischen Bestimmungsmethoden üblich, in gepufferter wässriger Lösung durchgeführt. Im allgemeinen erweist sich dabei ein pH-Bereich zwischen 6 und 10,5 als geeignet. Hinsichtlich der Art des Puffers bestehen keine Beschränkungen. Bevorzugt werden Glycinpuffer, Glycylglycinpuffer, Tris-Puffer und TRA-Puffer. Ferner wird die Umsetzung zweckmässig in Gegenwart von Magnesiumionen vorgenommen, beispielsweise in Gegenwart von Magnesiumchlorid.

Wie bereits erwähnt, eignet sich das erfindungsgemässe Verfahren nicht zur Bestimmung von Fructose selbst, sondern auch zur Bestimmung von anderen, Fructose enthaltenden Kohlehydraten, aus denen sich die Fructose quantitativ abspalten lässt, so dass die freigesetzte Fructose ein Mass für die Menge des Kohlehydrats darstellt. Zu derartigen Kohlehydraten gehören beispielsweise Disaccharide, Trisaccharide, Tetrasaccharide, Pentaosen und Polysaccharide. In der folgenden Tabelle sind Beispiele für derartige Fructoseeinheiten enthaltende Kohlehydrate aufgeführt unter Angabe ihrer Struktur, für die Spaltung geeigneter Enzyme und Vorkommen. Soweit keine spezifische Spaltungsenzyme zur Verfügung stehen, kann die Spaltung chemisch erfolgen, beispielsweise durch Säurehydrolyse nach üblichen Methoden. Ein Beispiel hierfür ist die Hydrolyse mit verdünnter Perchlorsäure.

Tabelle

| Name | Struktur | Spaltungsenzyme | Vorkommen |
|---|---|---|---|
| **Disaccharide** | | | |
| Xylosyl-Fructose | Xyl Fru | | |
| Furanose | Glu 1/3 Fru | | Bienenhonig |
| Maltulose | α-Glu 4 Fru | | Bienenhonig, Bier |
| Cellobiulose | β-Glu 4 Fru | | |
| Palatinose | Glu 6 Fru | α-Glucosidase | |
| Galsucrose | Gal-3-Fru | Emulsin | |
| Lactulose | Gal-4-Fru | Galactosidase | Arzneimittel |
| Planteobiose | Gal-6-Fru | | |
| Fructosyldesoxyglucose | Fru 1/6 Glu | | Tabak, Mais |
| Inulobiose | Fru 1/2 Fru | | |
| **Trisaccharide** | | | |
| Erlose | Glu 1/4 Glu 1/2 Fru | Invertase (Hefe) | |
| Glucosyl-Saccharose | Glu 1/6 Glu 1/2 Fru | | |
| Gentianose | Glu 1/2 Glu 1/2 Fru | Invertase (Hefe) liefert Gentiobiose und Fructose Emulsin liefert Saccharose und Glucose | |
| Umbelliferose | Gal 1/2 Glu 1/2 Fru | | |
| Lactosaccharose | Gal 1/4 Glu 1/2 Fru | | |
| Raffinose | Gal 1/6 Glu 1/2 Fru | Invertase | Melasse, Extrakte aus Zuckerrüben; stört Kristallisation der Saccharose |
| Kestose | Glu 1/2 Fru 1/2 Fru | | Bienenhonig |
| 6-Kestose | Glu 1/2 Fru 6/2 Fru | | Bienenhonig |
| Melizitose | Glu 1/3 Fru 2/1 Glu | α-Glucosidase | Manna |
| Planteose | Gal 1/6 Fru 1/2 Glu | | Tabak |
| **Tetrasaccharide** | | | |
| Fungitetraose | Glu 1/2 Fru 1/2 Fru 1/2 Fru | | |
| Stachyose | Gal 1/6 Gal 1/6 Glu 1/2 Fru | Invertase | Artischocken, Soja |
| Lychnose | Gal 1/6 Glu 1/2 Fru 1/1 Gal | | |
| Bisfurcose | Bis-(2/1 Fru 2/6 Fru)-Fructofuranosyl-saccharose | | Hafer |
| **Pentaosen** | | | |
| Verbascose | Gal 1/6 Gal 1/6 Gal 1/6 Glu 1/2 Fru | | Wollkraut, Wurzeln |
| **Polysaccharide** | | | |
| Inulin | Fru 1/2 $[Fru\ 1/2]_n$ 2/1 Glu (dazu ca, 6% Glucoseeinheiten in der Kette) | Invertase Inulase | Dahlienknollen Chicorée Artischocken |
| Irisin | " | | Iriswurzeln |
| Sitosin | " | | Weizenähren |
| Laevane | Fru 2/6 $[Fru\ 2/6]_n$ Fru 2/1 Glu | | Gräser, Halme |
| Phlein | Fru 2/6 $[Fru\ 2/6]_n$ Fru 2/1 Glu | | |
| Avenarin | " | | Haferhalme |
| Secalin | " | | Roggenstroh |
| Fructosan | Verzweigtes Polyfructosid [2/6-Verknüpfung] mit endständiger Glucose | | |

Ein weiterer Gegenstand der Erfindung ist ein Reagens zur Bestimmung von Fructose in Gegenwart von im Überschuss vorliegenden anderen Zuckern oder Polysacchariden und zur Bestimmung von Fructose enthaltenden Glykosiden, insbesondere in Gegenwart von im Überschuss vorliegenden anderen Zuckern oder Polysacchariden, welches dadurch gekennzeichnet ist, dass es UDPG, Saccharosesynthease, Puffersubstanz und ein System zur Bestimmung von UDP enthält.

In einer bevorzugten Ausführungsform dieses Reagens besteht das System zur Bestimmung von UDP aus Phosphoenolpyruvat, Pyruvatkinase, Lactatdehydrogenase und NADH.

Besonders günstige Ergebnisse bei Verwendung dieses bevorzugten Systems werden mit einem Reagens erhalten, welches
0,05 bis 10 U/ml Saccharosesynthetase,
0,05 bis 50 U/ml Pyrovatkinase,
0,05 bis 50 U/ml Lactatdehydrogenase,
1 bis 15 mMol/l UDPG,
0,1 bis 10 mMol/l Phosphoenolpyruvat,
0,1 bis 1 mMol/l NADH und
0,05 bis 0,5 Mol/l Puffer, pH 6 bis 10,5,
bezogen auf fertige Testlösung, enthält.

Die vorstehenden Konzentrationsangaben beziehen sich auf das fertige Testreagens in gelöster Form. Neben den angeführten Bestandteilen kann das Reagens noch Magnesiumsalze, Stabilisatoren, Aktivatoren für die Enzyme enthalten. Derartige Substanzen sind dem Fachmann bekannt und für enzymatische Reagenzien üblich. Das Reagens liegt vorzugsweise in Trockenform vor, beispielsweise in lyophilisiertem Zustand, da es in dieser Form haltbarer ist als in gelöstem Zustand. Desgleichen kann das Reagens einen festen, vorzugsweise blattförmigen oder streifenförmigen Träger aufweisen, welcher mit dem Reagens imprägniert ist, unter Bildung eines sogenannten «Teststreifens». Geeignet sind die für Teststreifen gebräuchlichen Träger, wie Papier, poröse Plastikfolien u.ä.

In einer weiteren Ausführungsform besteht das System zur Bestimmung von UDP aus einem Phosphatdonator, z.B. ATP, Formylphosphat oder Arginylphosphat, der entsprechenden Kinase, z.B. ATP-Kinase, Formiatkinase oder Argininkinase, und einem Reagens zur Bestimmung des entphosphorylierten Phosphatdonators.

Die Erfindung eignet sich grundsätzlich zur raschen und einfachen störungsfreien Bestimmung von Fructose jeglicher Herkunft. Besonders geeignet ist das Verfahren zur Bestimmung der Fructose in biologischen Substanzen, wie Lebensmitteln, Körperflüssigkeiten und dergleichen. Ein besonderer Vorteil ist darin zu sehen, dass die Bestimmung auch in Gegenwart grosser Überschüsse anderer Kohlehydrate durchführbar ist.

Die folgenden Beispiele erläutern die Erfindung weiter.

Die in den Beispielen und in der vorangehenden Beschreibung verwendeten Abkürzungen sind in der folgenden Liste zusammengefasst und erläutert:

| | |
|---|---|
| GOD | Glucoseoxidase |
| LDH | Lactatdehydrogenase |
| NAD | β-Nicotinamid-adenin-dinucleotid |
| NADH | β-Nicotinamid-adenin-dinucleotid, reduziert |
| PEP | Phosphoenolpyruvat |
| PK | Pyruvatkinase |
| Tra | Triäthanolamin |
| UDP | Uridin-5′-diphosphat |
| UDPG | Uridin-5′-diphosphoglucose |
| UTP | Uridin-5′-triphosphat. |

Beispiel 1

Bestimmung von Saccharose in reiner Lösung. Messtemperatur: 25 °C, Messwellenlänge: 365 nm, 1 cm Küvetten, Testvolumen: 3,34 ml.

| Ausgangslösung | Probe (ml) | Leerwert (ml) | Konzentration in der Inkubationslösung bzw. im Test |
|---|---|---|---|
| Citrat-Puffer, 0,32 Mol/l pH 4,6 | 0,2 | 0,2 | 0,2 Mol/l |
| Invertase (β-Fructosidase) 80 U/ml | 0,02 | 0,02 | 5 U/ml |
| Probelösung (ca. 1 mg Saccharose/ml) | 0,1 | – | ca. 1 mMol/l |

mischen, ca. 15 Minuten bei Raumtemperatur inkubieren, dann 2 Minuten bei 100 °C erhitzen. Zugabe von

| | | | |
|---|---|---|---|
| Tra-Puffer, pH 8,0 0,3 Mol/l | 1,0 | 1,0 | 89 mMol/l |
| NADH, 6,0 mMol/l | 0,1 | 0,1 | 0,18 mMol/l |
| PEP, 45 mMol/l | 0,1 | 0,1 | 1,3 mMol/l |
| UDPG, 73 mMol/l | 0,1 | 0,1 | 2,2 mMol/l |
| $MgCl_2$ und KCl, je 0,2 Mol/l | 0,1 | 0,1 | 6,0 mMol/l |
| PK/LDH, je 200 U/ml | 0,02 | 0,02 | je 1,2 U/ml |
| $H_2O$ | 1,4 | 1,5 | – |

Tabelle (Fortsetzung)

| Ausgangslösung | Probe (ml) | Leerwert (ml) | Konzentration in der Inkubationslösung bzw. im Test |
|---|---|---|---|
| mischen, Extinktion $E_1$ nach ca. 5 Minuten messen, dann starten mit | | | |
| Saccharosesynthetase 20 U/ml | 0,2 | 0,2 | 1,2 U/ml |

mischen, ca. 30 Minuten inkubieren, nach Stillstand der Extinktionsabnahme Extinktion der Probe gegen Extinktion des Leerwerts messen ($E_2$).

Berechnung von $\Delta E$ aus den Extinktionsdifferenzen. Aus der Differenz ergibt sich der Saccharosegehalt nach der Formel:

$$\text{Saccharose [mMol/1]} = \Delta E \times 9{,}82$$

Beispiel 2

Bestimmung von geringen Fructosekonzentrationen neben 200fachen Überschüssen an Glucosekonzentrationen. Messtemperatur: 25 °C, Messwellenlänge: 365 nm, 1 cm Küvetten, Testvolumen: 3,12 ml.

| Ausgangslösung | Probe (ml) | Leerwert (ml) | Konzentration im Test |
|---|---|---|---|
| Tra-Puffer, pH 8,0; 0,1 Mol/l | 1,0 | 1,0 | 33 mMol/l |
| NADH, 6,0 mMol/l | 0,1 | 0,1 | 0,2 mMol/l |
| PEP, 45 mMol/l | 0,1 | 0,1 | 1,5 mMol/l |
| UDPG, 73 mMol/l | 0,1 | 0,1 | 2,4 mMol/l |
| $MgCl_2$/KCl, je 0,2 Mol/l | 0,1 | 0,1 | je 6,7 mMol/l |
| PK/LDH, je 200 U/ml | 0,02 | 0,02 | je 1,3 U/ml |
| Glucose, 320 mMol/l | 0,1 | 0,1 | 10 mMol/l |
| $H_2O$ | 1,3 | 1,4 | – |
| Probelösung (ca. 0,5 mg Fructose/ml) | 0,1 | – | ca. 0,1 mMol/l |
| mischen, Extinktion $E_1$ nach ca. 5 Minuten messen, dann starten mit | | | |
| Saccharosesynthetase, 20 U/ml | 0,2 | 0,2 | 1,3 U/ml |

mischen, ca. 30 Minuten inkubieren, nach Stillstand der Extinktionsabnahme Extinktion der Probe und Extinktion des Leerwerts messen ($E_2$).

Berechnung von $\Delta E$ aus den Extinktionsdifferenzen. Aus der Differenz ergibt sich der Fructosegehalt der Probe nach der Formel:

$$\text{Fructose [mMol/l]} = \Delta E \times 9{,}18.$$

Beispiel 3

Bestimmung von Inulin im Serum.

Durch Hydrolyse mit Säure wird Inulin quantitativ zu Fructose hydrolysiert, welche dann bestimmt wird.

Hydrolysetemperatur: 80 °C, Messtemperatur: 25 °C

Messwellenlänge: 365 nm, 1 cm Küvetten, Testvolumen: 3,22 ml.

| Ausgangslösung | Probe (ml) | Leerwert (ml) | Konzentration in der Inkubationslösung |
|---|---|---|---|
| Vollblut (mit ca. 0,5 g Inulin/l) | 0,1 | – | ca. 0,28 mMol/l |
| Perchlorsäure, 0,3 Mol/l | 1,0 | 1,0 | 0,27 Mol/l |
| mischen und ca. 15 Minuten bei ca. 80 °C halten, abkühlen und zentrifugieren. Vom Zentrifugat entnehmen: | | | |

| Ausgangslösung | Probe (ml) | Leerwert (ml) | Konzentration im Test |
|---|---|---|---|
| Überstand | 0,1 | 0,1 | |
| Tra-Puffer, pH 8,0; 0,3 Mol | 1,0 | 1,0 | 93 mMol/l |
| NADH, 6,0 mMol/l | 0,1 | 0,1 | 0,19 mMol/l |
| UDPG, 73 mMol/l | 0,1 | 0,1 | 2,3 mMol/l |
| PEP, 45 mMol/l | 0,1 | 0,1 | 1,5 mMol/l |
| $MgCl_2$/KCl, je 0,2 Mol/l | 0,1 | 0,1 | 6,2 mMol/l |
| PK/LDH, je 200 U/ml | 0,02 | 0,02 | 1,3 U/ml |
| $H_2O$ | 1,4 | 1,5 | – |
| mischen, Extinktion $E_1$ nach ca. 5 Minuten messen, dann starten mit | | | |
| Saccharosesynthetase, 20 U/ml | 0,2 | 0,2 | 1,2 U/ml |

mischen, ca. 30 Minuten inkubieren, nach Stillstand der Extinktionsabnahme Extinktion der Probe und des Leerwerts ($E_2$) messen.

Berechnung von ΔE aus den Extinktionsdifferenzen. Aus der Differenz ergibt sich der Inulingehalt nach der Formel:

$$\text{Inulin [mMol/l]} = \Delta E \times \frac{3{,}22 \cdot (1{,}0 + 0{,}1 \cdot 0{,}81^{a}) \cdot 1{,}04^{b}}{3{,}4 \cdot 0{,}1} = \Delta E \times 10{,}65$$

a) Faktor, aus der Volumenkontraktion des Blutes nach Enteiweissung berechnet.

b) Faktor, welcher den durchschnittlichen Glucosegehalt von 4% in Inulin berücksichtigt.

Bei Anwesenheit von freier Fructose im Blut muss diese in einem getrennten Testansatz ohne Perchlorsäurebehandlung bestimmt werden.

**Patentansprüche**

1. Verfahren zur Bestimmung von Fructose in Gegenwart von im Überschuss vorliegenden anderen Zuckern oder Polysacchariden und zur Bestimmung von Fructose enthaltenden Glykosiden, insbesondere in Gegenwart von im Überschuss vorliegenden anderen Zuckern oder Polysacchariden, dadurch gekennzeichnet, dass die gegebenenfalls zuerst aus dem Glykosid in Freiheit gesetzte Fructose mit UDPG und Saccharosesynthetase umgesetzt wird unter Bildung von UDP, welches in an sich bekannter Weise bestimmt wird.

2. Verfahren nach Anspruch 1, dadurch gekennzeichnet, dass die Umsetzung mit UDPG bei einem pH-Wert zwischen 6 und 10,5 durchgeführt wird.

3. Verfahren nach Anspruch 1 oder 2, dadurch gekennzeichnet, dass als Fructose enthaltendes Glykosid Saccharose bestimmt und die Fructose daraus durch Invertase freigesetzt wird.

4. Verfahren nach einem der Ansprüche 1 bis 3, dadurch gekennzeichnet, dass als Fructose enthaltendes Glykosid Inulin bestimmt und die Fructose durch Säurehydrolyse daraus freigesetzt wird.

5. Verfahren nach einem der vorhergehenden Ansprüche, dadurch gekennzeichnet, dass die Bestimmung von gebildetem UDP durch Umsetzung mit Phosphoenolpyruvat, Pyruvatkinase und Lactatdehydrogenase in Gegenwart von NADH erfolgt und die Abnahme an NADH gemessen wird.

6. Reagenz zur Bestimmung von Fructose in Gegenwart von im Überschuss vorliegenden anderen Zuckern oder Polysacchariden und zur Bestimmung von Fructose enthaltenden Glykosiden, insbesondere in Gegenwart von im Überschuss vorliegenden anderen Zuckern oder Polysacchariden, dadurch gekennzeichnet, dass es UDPG, Saccharosesynthetase, Puffersubstanz und ein System zur Bestimmung von UDP enthält.

7. Reagens nach Anspruch 6, dadurch gekennzeichnet, dass das System zur Bestimmung von UDP aus Phosphoenolpyruvat, Pyruvatkinase, Lactatdehydrogenase und NADH besteht.

8. Reagens nach Anspruch 7, dadurch gekennzeichnet, dass es
0,05 bis 10 U/ml Saccharosesynthetase,
0,05 bis 50 U/ml Pyruvatkinase,
0,05 bis 50 U/ml Lactatdehydrogenase,
1 bis 15 mMol/1 UDPG,
0,1 bis 10 mMol/l Phosphoenolpyruvat,
0,1 bis 1 mMol/l NADH und
0,05 bis 0,5 Mol/l Puffer, pH 6 bis 10,5, bezogen auf fertige Testlösung, enthält.

9. Reagens nach Anspruch 6, dadurch gekennzeichnet, dass das System zur Bestimmung von UDP aus einem Phosphatdonator, seiner spezifischen Kinase und einem Reagens zur Bestimmung des entphosphorylierten Phosphatdonators besteht.

10. Reagens nach einem der Ansprüche 6 bis 9, dadurch gekennzeichnet, dass es zusätzlich Magnesiumsalz oder/und Stabilisatoren oder/und Aktivatoren oder/und ein festes blatt- oder streifenförmiges Trägermaterial enthält.

## Claims

1. Process for the determination of fructose in the presence of other sugars or polysaccharides present in excess and for the determination of fructose-containing glycosides, especially in the presence of other sugars or polysaccharides present in excess, characterised in that the fructose, possibly first liberated from the glycoside, is reacted with UDPG and saccharose synthetase with the formation of UDP which is determined in known manner.

2. Process according to claim 1, characterised in that the reaction with UDPG is carried out at a pH value between 6 and 10.5.

3. Process according to claim 1 or 2, characterised in that, as fructose-containing glycoside, saccharose is determined and the fructose is liberated therefrom by invertase.

4. Process according to one of claims 1 to 3, characterised in that as fructose-containing glycoside, inulin is determined and the fructose is liberated therefrom by acid hydrolysis.

5. Process according to one of the preceding claims, characterised in that UDP formed is determined by reaction with phosphoenol pyruvate, pyruvate kinase and lactate dehydrogenase in the presence of NADH and the decrease of NADH is measured.

6. Reagent for the determination of fructose in the presence of other sugars or polysaccharides present in excess and for the determination of fructose-containing glycosides, especially in the presence of other sugars or polysaccharides present in excess, characterised in that it contains UDPG, saccharose synthetase, buffer substance and a system for the determination of UDP.

7. Reagent according to claim 6, characterised in that the system for the determination of UDP consists of phosphoenol pyruvate, pyruvate kinase, lactate dehydrogenase and NADH.

8. Reagent according to claim 7, characterised in that it contains
0.05 to 10 U/ml saccharose synthetase,
0.05 to 50 U/ml pyruvate kinase,
0.05 to 50 U/ml lactate dehydrogenase,
1 to 15 mMol/l UDPG,
0.1 to 10 mMol/l phosphoenol pyruvate,
0.1 to 1 mMol/l NADH and
0.05 to 0.5 Mol/l buffer, pH 6 to 10.5,
referred to the final test solution.

9. Reagent according to claim 6, characterised in that the system for the determination of UDP consists of a phosphate donor, its specific kinase and a reagent for the determination of the dephosphorylated phosphate donor.

10. Reagent according to one of claims 6 to 9, characterised in that it additionally contains magnesium salt and/or stabilisers and/or activators and/or a solid leaf- or strip-shaped carrier material.

## Revendications

1. Procédé de dosage du fructose en présence d'autres sucres ou de polysaccharides présents en excès et de dosage de glycosides contenant du fructose, en particulier en présence d'autres sucres ou polysaccharides présents en excès, caractérisé en ce que le fructose éventuellement mis d'abord en liberté à partir du glycoside, est mis à réagir avec de l'UDPG et de la saccharosesynthétase avec formation d'UDP, lequel est dosé d'une manière connue en soi.

2. Procédé suivant la revendication 1, caractérisé en ce que la réaction avec l'UDPG est effectuée à une valeur de pH entre 6 et 10,5.

3. Procédé suivant la revendication 1 ou 2, caractérisé en ce que comme glycoside contenant du fructose, on dose le saccharose et en ce que le fructose est libéré de celui-ci par l'invertase.

4. Procédé suivant l'une des revendications 1 à 3, caractérisé en ce que comme glycoside contenant du fructose, on dose l'inuline et en ce que le fructose est libéré de celle-ci par hydrolyse acide.

5. Procédé suivant l'une des revendications précédentes, caratérisé en ce que le dosage de l'UDP formé s'effectue par réaction avec le phosphoénolpyruvate, la pyruvatekinase et la lactactedeshydrogénase en présence de NADH et en ce qu'on mesure la diminution du NADH.

6. Réactif pour le dosage du fructose en présence d'autres sucres ou de polysaccharides présents en excès et pour le dosage de glycosides contenant du fructose, en particulier en présence d'autres sucres ou polysaccharides présents en excès, caractérisé en ce qu'il contient de l'UDPG, de la saccharosesynthétase, une substance tampon et un système de dosage de l'UDP.

7. Réactif suivant la revendication 6, caractérisé en ce que le système de dosage de l'UDP se compose de phosphoénolpyruvate, de pyruvatekinase, de lactatedeshydrogénase et de NADH.

8. Réactif suivant la revendication 7, caractérisé en ce qu'il contient
0,05 à 10 U/ml de saccharosesynthétase,
0,05 à 50 U/ml de pyruvatekinase,
0,05 à 50 U/ml de lactatedeshydrogénase,
1 à 15 mmole/l de UDPG,
0,1 à 10 mmole/l de phosphoénolpyruvate,
0,1 à 1 mmole/l de NADH et
0,05 à 0,5 mole/l de tampon, pH 6 à 10,5,
par rapport à la solution d'essai terminée.

9. Réactif suivant la revendication 6, caractérisé en ce que le système de dosage de l'UDP se compose d'un donneur de phosphate, de sa kinase spécifique et d'un réactif pour le dosage du donneur de phosphate déphosphorylé.

10. Réactif suivant l'une des revendications 6 à 9, caractérisé en ce qu'il contient en outre du sel de magnésium ou/et des stabilisants ou/et des activateurs ou/et une matière de support solide sous forme de feuille ou de ruban.